(19) Europäisches Patentamt
European Patent Office
Office européen des brevets

(11) Publication number: **0 675 109 A1**

# (12) EUROPEAN PATENT APPLICATION published in accordance with Art. 158(3) EPC

(21) Application number: **94903029.0**

(51) Int. Cl.[6]: **C07D 209/14**, //A61K31/40

(22) Date of filing: **21.12.93**

(86) International application number: **PCT/JP93/01845**

(87) International publication number: **WO 94/14769 (07.07.94 94/15)**

(30) Priority: **21.12.92 JP 340448/92**
**05.03.93 JP 44927/93**

(43) Date of publication of application: **04.10.95 Bulletin 95/40**

(84) Designated Contracting States: **AT BE CH DE DK ES FR GB GR IE IT LI LU MC NL PT SE**

(71) Applicant: **TAISHO PHARMACEUTICAL CO. LTD**
**24-1 Takata 3-chome**
**Toshima-ku**
**Tokyo 171 (JP)**

(72) Inventor: **IKEMOTO, Tomoyuki, Taisho Pharmaceutical Co., Ltd.**
**24-1, Takata 3-chome,**
**Toshima-ku**
**Tokyo 171 (JP)**
Inventor: **HORIGUCHI, Akiyo, Taisho Pharmaceutical Co., Ltd.**
**24-1, Takata 3-chome,**
**Toshima-ku**
**Tokyo 171 (JP)**
Inventor: **KAWASHIMA, Yutaka, Taisho Pharmaceutical Co., Ltd.**
**24-1, Takata 3-chome,**
**Toshima-ku**
**Tokyo 171 (JP)**
Inventor: **HATAYAMA, Katsuo, Taisho Pharmaceutical Co., Ltd.**
**24-1, Takata 3-chome,**
**Toshima-ku**
**Tokyo 171 (JP)**

(74) Representative: **Kraus, Walter, Dr. et al**
**Patentanwälte Kraus, Weisert & Partner**
**Thomas-Wimmer-Ring 15**
**D-80539 München (DE)**

## (54) N-SUBSTITUTED INDOLE DERIVATIVE.

(57) The object is to provide a compound having potent and persistent effects of lowering blood pressure and ameliorating urination disorder by blocking α1-adrenergic receptor. An N-substituted indole derivative represented by general formula (I) and an acid-addition salt thereof, wherein $R^1$ represents alkyl, or phenyl which may be substituted by halogen, alkyl or alkoxy; $R^2$ represents phenyl which may be substituted by one or two groups selected from among halogen, alkyl, alkoxy and 1-piperidyl, or 2-pyridyl which may be substituted by one or two groups selected from among halogen, alkyl and alkoxy; and n represents an integer of 2 to 5.

EP 0 675 109 A1

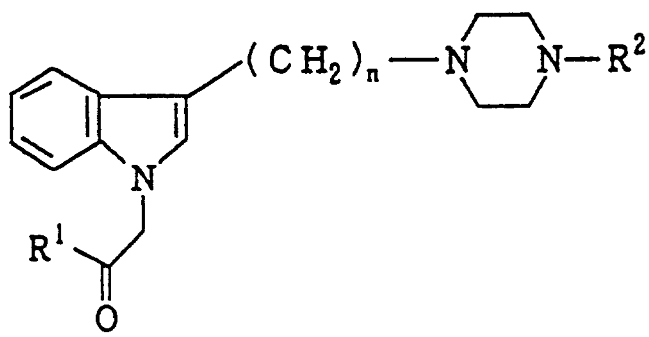

(I)

## TECHNICAL FIELD

This invention relates to an N-substituted indole derivative which exhibits a depressor activity and a urination disorder-relieving activity by blocking the α1-adrenergic receptor.

## BACKGROUND ART

α1-Adrenergic receptor blocking agents, which have been employed mainly as a hypotensive drug, are characterized by being usable in treating cardiac function depression and renal function depression without lowering cardiac output or organ perfusion. In recent years, these α1-adrenergic receptor blocking agents have been also used in relieving urination disorders. There have been known compounds having a depressor activity (prazosin, doxazosin, urapidil, etc.) and compounds having a urination disorder-relieving activity (prazosin, urapidil, etc.). However, each of these compounds differs from the compound of the present invention in the fundamental structure.

An object of the present invention is to provide a compound which strongly and persistently shows a depressor activity and a urination disorder-relieving activity by blocking the α1-adrenergic receptor.

## DISCLOSURE OF THE INVENTION

As a result of extensive investigation to solve the above-mentioned problems, the present inventors have found out that a specific N-substituted indole derivative shows a potent depressor activity and a potent urination disorder-relieving activity by blocking the α1-adrenergic receptor, thus completing the present invention.

Accordingly, the present invention relates to an N-substituted indole derivative represented by the following formula:

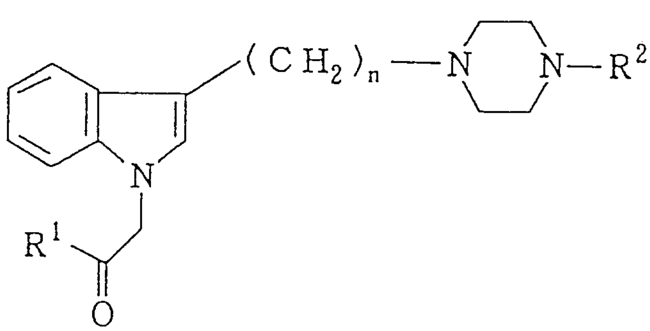

(I)

wherein $R^1$ represents an alkyl group having 1 to 8 carbon atoms, a phenyl group, or a phenyl group substituted by a halogen atom, an alkyl group having 1 to 4 carbon atoms or an alkoxy group having 1 to 4 carbon atoms, $R^2$ represents a phenyl group, a phenyl group substituted by one or two substituents selected from a halogen atom, an alkyl group having 1 to 4 carbon atoms, an alkoxy group having 1 to 4 carbon atoms and a piperidino group, a 2-pyridyl group, or a 2-pyridyl group substituted by one or two substituents selected from a halogen atom, an alkyl group having 1 to 4 carbon atoms and an alkoxy group having 1 to 4 carbon atoms; and n is an integer of from 2 to 5; and an acid addition salt thereof.

The term "alkyl group" as used herein means a linear or branched alkyl group. The term "alkoxy group" means a linear or branched alkoxy group. The term "halogen atom" means a fluorine, chlorine, bromine or iodine atom. The term "acid addition salt of the compound of the formula (I)" means a salt formed by the addition of an inorganic or organic acid. Examples of the inorganic or organic acid to be used herein involve hydrochloric acid, oxalic acid, hydrobromic acid, sulfuric acid, phosphoric acid, formic acid, acetic acid, propionic acid, glycolic acid, fumaric acid, succinic acid, tartaric acid, ascorbic acid, salicylic acid, lactic acid, malic acid, methanesulfonic acid and p-toluenesulfonic acid, though the present invention is not restricted thereto. When the phenyl or 2-pyridyl group represented by $R^2$ has two substituents, these substituents may be either one and the same or different from each other.

A particularly preferable compound of the present invention is 3-[2-(4-(2-methoxyphenyl)piperadinyl)-ethyl]-1-(2-oxobutyl)indole.

The compound of the present invention can be prepared by, for example, the following method.

Namely, an indole derivative represented by the formula:

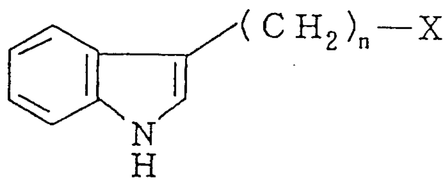

wherein n is as defined above; and X represents a halogen atom or a methanesulfonyloxy group; is first reacted with a compound represented by the formula:

$Y-CH_2-CO-R^1$ (II)

wherein Y represents a halogen atom or a methanesulfonyloxy group; and $R^1$ is as defined above; in a solvent in the presence of a base (for example, potassium carbonate, triethylamine) to obtain a compound represented by the formula:

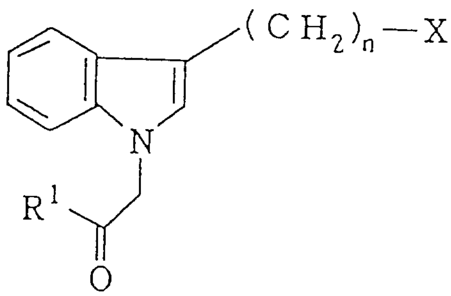

wherein $R^1$, n and X are each as defined above.

Examples of the solvent to be used herein include a nitrile solvent (for example, acetonitrile) and a halogenated solvent (for example, dichloromethane or chloroform). The reaction temperature ranges from 0 to 100 °C and the reaction time ranges from 10 minutes to 48 hours.

Next, the compound obtained above is reacted with a compound represented by the formula:

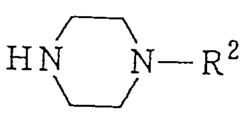

(III)

wherein $R^2$ is as defined above;
in a solvent in the presence of a base (for example, potassium carbonate or triethylamine). Thus the compound of the formula (I) of the present invention can be obtained.

Examples of the solvent to be used herein include a nitrile solvent (for example, acetonitrile) and a halogenated solvent (for example, dichloromethane or chloroform). The reaction temperature ranges from 0 to 100 °C and the reaction time ranges from 10 minutes to 48 hours.

## BEST MODE FOR CARRYING OUT THE INVENTION

To further illustrate the present invention in greater detail, the following Examples and Test Examples will be given.

(Example 1)

Production of 3-[2-(4-(2-methoxyphenyl)piperadinyl)ethyl]-1-(2-oxobutyl)indole (compound 1)

(1) 3.00 g of 3-(2-bromoethyl)indole and 2.22 g of 1-bromo-2-butanone were dissolved in 30 ml of acetonitrile. After adding 1.85 g of potassium carbonate, the mixture was heated under reflux for 4 hours. The solvent in the reaction mixture was evaporated under reduced pressure and water was added to the residue. Then the mixture was extracted with ethyl acetate, washed with water and a saturated aqueous solution of sodium chloride, dried over magnesium sulfate and filtered followed by concentration. The residue was subjected to silica gel column chromatography (developing solvent; hexane : ethyl acetate = 12 : 1 to 10 : 1) to give 3.03 g of 3-(2-bromoethyl)-1-(2-oxobutyl)indole

(2) 3.02 g of the 3-(2-bromoethyl)-1-(2-oxobutyl)indole obtained in the above (1) and 2.50 g of 2-methoxyphenylpiperazine were dissolved in 80 ml of acetonitrile. After adding 1.32 g of triethylamine, the mixture was heated under reflux for 6 hours. The solvent in the reaction mixture was evaporated under reduced pressure and water was added to the residue. Then the mixture was extracted with ethyl acetate, washed with water and a saturated aqueous solution of sodium chloride, dried over magnesium sulfate and filtered followed by concentration. The residue was subjected to silica gel column chromatography (developing solvent; hexane: ethyl acetate = 4 : 1 to 2 : 1). The crude product thus obtained was recrystallized from ethyl acetate to give 3.1 g of the title compound.

m.p.: 132 - 133 °C.

(Example 2)

Production of 3-[2-(4-(2-methoxyphenyl)piperadinyl)ethyl]-1-(2-oxobutyl)indole dihydrochloride

90 mg of the Compound 1 obtained in the above Example 1 was dissolved in a mixture of ethanol with ether. After adding a 4 N solution of hydrogen chloride in ethyl acetate, the precipitate was recovered by filtration and recrystallized from ethanol to give 91 mg of the title compound.

m.p.: 145 - 146 °C.

(Examples 3 to 21)

Compounds listed in the following Table 1 were obtained in the substantially same manner as that of Example 1 (if necessary, performing the substantially same procedure as in Example 2) except for using each of the corresponding compounds of the formula (II) in place of the 1-bromo-2-butanone used in Example 1 (1) and each of the corresponding compounds of the formula (III) in place of the 2-methoxyphenylpiperazine used in Example 1 (2).

Table 1

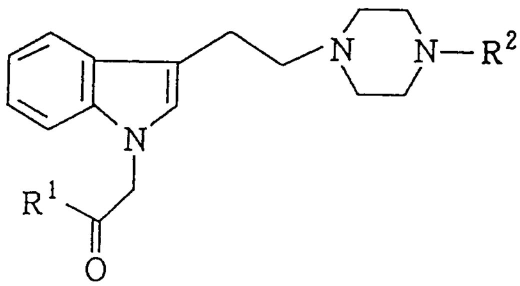

| Ex. No. | $R^1$ | $R^2$ | Salt | m.p. (°C) |
|---|---|---|---|---|
| 3 | ethyl | 6-methyl-2-pyridyl | - | 126-128 |
| 4 | ethyl | 2,3-dimethylphenyl | hydrochloride | 228-231Δ |
| 5 | ethyl | 3-fluorophenyl | hydrochloride | 196-198 |
| 6 | ethyl | 2-piperidinophenyl | - | 145-147 |
| 7 | ethyl | 2,5-dimethoxy-phenyl | dihydro-chloride | 159-162 |
| 8 | ethyl | 4-fluorophenyl | hydrochloride | 186-189Δ |
| 9 | methyl | 2-methoxyphenyl | - | 186-188 |
| 10 | methyl | 2-methoxyphenyl | dihydro-chloride | 192-195 |
| 11 | methyl | 2,3-dimethylphenyl | hydrochloride | 217-220Δ |
| 12 | methyl | 2-chlorophenyl | oxalate | 106-108 |
| 13 | methyl | 2-isopropylphenyl | oxalate | 186-188 |
| 14 | propyl | 2-methoxyphenyl | - | 149-151 |
| 15 | isopropyl | 2-methoxyphenyl | dihydro-chloride | 189-192Δ |
| 16 | butyl | 2-methoxyphenyl | - | 121-123 |
| 17 | t-butyl | 2-methoxyphenyl | dihydro-chloride | 207-209Δ |

| | | | | |
|---|---|---|---|---|
| 18 | isobutyl | 2-methoxyphenyl | - | 105-106 |
| 19 | phenyl | 2-methoxyphenyl | dihydro-chloride | 174-177 |
| 20 | 3-methoxy-phenyl | 2-methoxyphenyl | dihydro-chloride | 157-159 |
| 21 | 4-chloro-phenyl | 2-methoxyphenyl | - | 236-240Δ |

Note: Δ means undergoing decomposition.

(Test Example 1)

[α1-Adrenergic receptor binding test]

The α1-adrenergic receptor binding reaction was performed in accordance with the method of Greengrass and Bremner [Eur. J. Pharmacol., 55, 323 (1979)].

A rat was decapitated, and the brain was taken out and homogenized with 30 volumes of 50 Mm Tris hydrochloride (Ph 7.4). The homogenate was centrifuged at 1,000 x g for 5 minutes and the supernatant was further centrifuged at 48,000 x g for 20 minutes. The precipitate thus obtained was suspended in 50 mM Tris hydrochloride (pH 7.4) and recentrifuged at 48,000 x g for 20 minutes. The obtained precipitate was suspended in 50 mM Tris hydrochloride (pH 7.4) in such a manner as to give a concentration of 1.0 mg/ml protein. Thus an α1-adrenergic receptor preparation was obtained.

To 1.0 ml portions of this receptor preparation were added 0.6 nM [$^3$H] prazosin and specimens at various concentrations, and the mixture was allowed to react at 25 °C for 30 minutes.

After the completion of the reaction, each reaction mixture was subjected to a rapid filtration through a glass filter (Whatman GF/B) and the filter was washed with 3 ml portions of 50 mM tris hydrochloride (pH 7.4) thrice. The radioactivity on the filter was measured with a liquid scintillation counter.

The difference between the radioactivity in the absence of any specimen and the radioactivity obtained in the presence of 10 µM of prazosin was referred to as the specific binding of the control. The ratio of the radioactivity obtained at the time of adding each specimen to the control radioactivity was determined and plotted against the specimen concentration. Then the fifty percent inhibitory concentration ($IC_{50}$) of each specimen was calculated by the curve fitting method with the use of a computer.

The results are shown in Table 2.

Table 2

| Specimen | $IC_{50}$ (nM) |
|---|---|
| compound 1 | 7.75 |
| compound 4 | 3.93 |
| compound 8 | 5.13 |
| compound 14 | 0.801 |
| compound 15 | 0.679 |
| urapidil | 741 |
| Note: The compounds 1, 4, 8, 14 and 15 given in the table are the products produced respectively in the above Examples 1, 4, 8, 14 and 15 (the same will apply in the following Test Examples). | |

(Test Example 2) [Depressor activity test]

The depressor activity test was performed in accordance with the method described in Journal of Physiology, 448, 307 - 320 (1992).

Under pentobarbital anesthesia, a catheter for detecting and transmitting the blood pressure was embedded in a branch of the femoral artery of a dog (weighing 10 kg, beagle, each group having 4 animals). When the animal restored from the postoperative aggression, a powder of the compound 1 encapsulated in a cellulose capsule (3 mg/kg) was orally administered to the animal, which had been fasted, without anesthesia or restriction.

Via the catheter embedded in the body of the beagle, the cardiac rate was measured from the blood pressure and the blood pressure pulse wave in accordance with the telemetry method. The blood pressure lowering effect attained the maximum level 30 minutes after the administration and the blood pressure was lowered by about 12 mmHg. This depressor activity persisted even 24 hours after the administration. The administration of this compound somewhat lowered the cardiac rate and thus the refractive frequent pulse accompanying the depression was sufficiently suppressed.

(Test Example 3) [Urination disorder-relieving activity test]

The urination disorder-relieving activity test was performed in accordance with the method described in FEDERATION PROCEEDINGS, 45 (11), (1986).

Under pentobarbital anesthesia, the prostate gland of a male dog (weighing 7 to 10 kg, beagle, each group having 3 animals) was taken out. Then a preparation of the smooth muscle of the beagle prostate gland thus taken out was prepared in a conventional manner and the isometric tensile strength was measured by Magnus' method. After incubating for 60 to 90 minutes, phenylephrine ($10^{-7}$ to $10^{-4}$ M) was accumulatively administered to give a control dose/reaction curve. After washing away the phenylephrine, the preparation was treated with a specimen ($10^{-8}$ to $10^{-6}$ M) for 15 minutes. In the presence of the specimen, phenylephrine ($10^{-7}$ to $10^{-3}$ M) was accumulatively administered again and a dose/reaction curve was obtained in the same manner. Based on these two dose/reaction curves, the $IC_{50}$ of each specimen was calculated.

The results are shown in Table 3.

Table 3

| Specimen | $IC_{50}$ (nM) |
|---|---|
| compound 1 | 1.4 |
| urapidil | 53.7 |

## INDUSTRIAL APPLICABILITY

As shown in the above Test Examples, the compound of the present invention blocks the $\alpha$1-adrenergic receptor and exhibits a potent depressor activity and a potent urination disorder-relieving activity. Thus it is usable as a hypotensive drug or a remedy for urination disorders.

For these purposes, the compound of the present invention can be processed, together with pharmaceutically acceptable additives, into preparations suitable for the administration and administered either orally or parenterally (for example, intravenously). Examples of the preparation for oral administration include solid preparations such as tablets, granules and capsules, and liquid preparations such as solutions, fat-emulsions and liposomes. Examples of the preparation for intravenous administration include aqueous solutions, non-aqueous solutions, emulsions, liposomes and suspensions.

Examples of the additives for producing these preparations include fillers such as crystalline cellulose, lactose, corn starch and mannitol; lubricants such as magnesium stearate and talc; binders such as hydrxypropylcellulose and polyvinylpyrrolidone; disintegrating agents such as carboxymethylcellulose calcium; fluidity improvers such as light silicic anhydride; solvents such as distilled water for injection, physiological saline and Ringer solution; preservatives such as methyl parahydroxybenzoate and propyl parahydroxybenzoate; emulsifiers such as gum arabic and lecithin; and surfactants such as Tween and Span.

The dose of the compound of the present invention may vary over a wide range depending on the age, sex, body weight and severity of the symptom of a patient and the judgement of a physician. In the case of a standard adult, the daily dose of the compound of the present invention employed as a remedy for urination disorders ranges from 0.1 to 50 mg, while its daily dose employed as a hypotensive drug ranges from 1 to 200 mg. If necessary, the compound may be administered in such a daily dose one to several portions per day.

## Claims

**1.** An N-substituted indole derivative represented by the following formula:

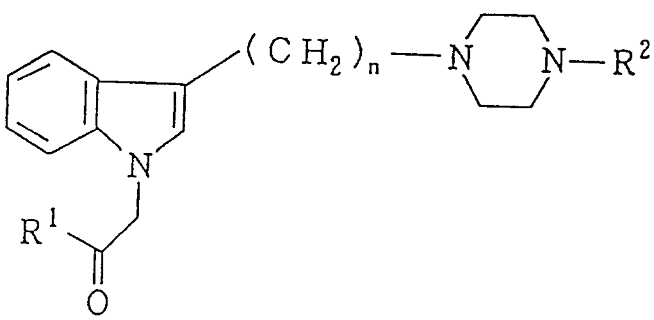

(I)

wherein $R^1$ represents an alkyl group having 1 to 8 carbon atoms, a phenyl group, or a phenyl group substituted by a halogen atom, an alkyl group having 1 to 4 carbon atoms or an alkoxy group having 1 to 4 carbon atoms; $R^2$ represents a phenyl group, a phenyl group substituted by one or two substituents selected from a halogen atom, an alkyl group having 1 to 4 carbon atoms, an alkoxy group having 1 to 4 carbon atoms and a piperidino group, a 2-pyridyl group , or a 2-pyridyl group substituted by one or two substituents selected from a halogen atom, an alkyl group having 1 to 4 carbon atoms and an alkoxy group having 1 to 4 carbon atoms; and n is an integer of from 2 to 5;
and an acid addition salt thereof.

**INTERNATIONAL SEARCH REPORT**

International application No. PCT/JP93/01845

**A. CLASSIFICATION OF SUBJECT MATTER**

Int. $Cl^5$ C07D209/14//A61K31/40

According to International Patent Classification (IPC) or to both national classification and IPC

**B. FIELDS SEARCHED**

Minimum documentation searched (classification system followed by classification symbols)

Int. $Cl^5$ C07D209/14//A61K31/40

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| A | JP, A, 2-225460 (H. Lundbeck A/S), September 7, 1990 (07. 09. 90) & EP, A, 376607 & US, A, 5002948 | 1 |
| A | JP, A, 2-85277 (Bristol-Myers Co.), March 26, 1990 (26. 03. 90) & EP, A, 345808 & US, A, 4954502 | 1 |
| A | Chemical Abstracts, Vol. 115, No. 1, 1991, abstract No. 8711w, Russo, F. et. al.: "Pyrimido [5, 4-b] indole derivatives 1, A new class of potent and selective α1 adrenoceptor ligands." & J. Med. Chem., 1991, 34(6), 1850-4 | 1 |
| A | Chemical Abstracts, Vol. 109, No. 17, 1988, abstract No. 149556n, Takebayashi et. a.: "3-(1-piperazinylalkyl)-2-thioxoguinazolin-4-one derivatives as selective. α1 blockers and a process for preparing them." & JP, A, 62-258369 (Nippon Soda Co., Ltd.), | 1 |

[X] Further documents are listed in the continuation of Box C. [ ] See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| March 1, 1994 (01. 03. 94) | March 22, 1994 (22. 03. 94) |
| Name and mailing address of the ISA/ Japanese Patent Office | Authorized officer |
| Facsimile No. | Telephone No. |

**INTERNATIONAL SEARCH REPORT**

International application No.
PCT/JP93/01845

C (Continuation). DOCUMENTS CONSIDERED TO BE RELEVANT

| Category* | Citation of document, with indication, where appropriate, of the relevant passages | Relevant to claim No. |
|---|---|---|
| | November 10, 1987 (10. 11. 87) | |
| A | JP, A, 1-93578 (Hoechst-Roussel Pharmaceuticals, Inc.), April 12, 1989 (12. 04. 89) & EP, A, 299349 | 1 |